# FASCICULE DE BREVET EUROPEEN

(11) **EP 0 901 381 B1**
(45) Date de publication et mention de la délivrance du brevet: **19.03.2003**
(21) Numéro de dépôt: 97919597.1
(22) Date de dépôt: 09.05.1997
(51) Int. Cl.: A61L 11/00, A61L 2/08, B09B 3/00

(54) **PROCEDE ET APPAREIL POUR STERILISER LES DECHETS MEDICAUX ET VETERINAIRES**
VERFAHREN UND VORRICHTUNG ZUM STERILISIEREN VON MEDIZINISCHEN UND VETERINÄREN ABFÄLLEN
METHOD AND APPARATUS FOR STERILISING MEDICAL AND VETERINARY WASTES

(30) Priorité: 17.05.1996 CH 124896
(43) Date de publication de la demande: 17.03.1999
(73) Titulaire: RIMM TECHNOLOGIES N.V., Curaçao (AN)
(72) Inventeur: KONGMARK, Nils, E., A., F-01220 Sauverny (FR)
(74) Mandataire: Moinas, Michel
(86) Numéro de dépôt international: IB9700532
(87) Numéro de publication internationale: WO97044069

(56) Documents cités:
- EP-A- 0 649 661
- EP-A- 0 672 426
- WO-A-91/15247
- WO-A-92/00764
- WO-A-92/04920
- WO-A-95/14496
- FR-A- 2 115 951

## Description

La présente invention se rapporte à un procédé et un appareil pour la stérilisation de déchets médicaux et vétérinaires en vue de leur élimination sans risque pour l'environnement.

Le traitement et l'élimination des déchets médicaux et vétérinaires est un problème d'une acuité croissante, notamment pour les hôpitaux et cliniques. Tous les moyens de traitement connus sont basés sur l'enlèvement des déchets du lieu où ils ont été créés pour les stocker dans un endroit où ils peuvent être considérés comme inoffensifs. Le problème est que ces déchets peuvent entraîner des contaminations durant leur transport, pas uniquement parce que les moyens de confinement lors du transport peuvent être déficients, mais par simple facteur humain. La meilleure méthode consisterait donc à stériliser les déchets infectieux, qui représentent environ 20% du total des déchets médicaux et vétérinaires, sur place, là où ils sont produits. Néanmoins, aucune solution satisfaisante de ce type n'existe à ce jour.

Le brevet WO 92/04920 décrit une méthode de traitement des déchets médicaux et l'appareil mettant en oeuvre cette méthode. Les déchets sont désintégrés et désinfectés par des radiations de fréquence radio amenant ces déchets à une température d'au moins 100°C. Les fréquences utilisées se situent entre 5 et 100 Megahertz, les fréquences plus élevées n'étant pas recommandées car elles ne pénètrent pas assez les déchets. Mais ce procédé n'a pas lieu sur place, les déchets étant amenés dans des containers hermétiquement clos pour être traités.

L'invention prétend résoudre ce problème en proposant un procédé pour le traitement des déchets médicaux et vétérinaires sur site, par stérilisation, comprenant les étapes consistant, dans l'ordre, à :
préparer les déchets en vue de leur manutention ultérieure et pour leur traitement subséquent par chauffage électromagnétique,
diviser. les déchets en charges. individuelles ou lots homogènes et de tailles sensiblement égales,
stériliser les déchets en lots par une source de chaleur générée par voie électromagnétique, et
véhiculer aseptiquement les déchets stérilisés hors de la zone de traitement et à reprendre ceux-ci sans exposition à l'extérieur, en vue de leur transport.

Le procédé trouve un intérêt tout particulier dans le cadre de l'élimination des déchets infectieux, mais il s'applique également aux autre déchets, de sorte que, à moins que ces derniers représentent la quasi-totalité des déchets par rapport aux déchets infectieux, il n'y aura généralement pas lieu de séparer préalablement les déchets infectieux des déchets non infectieux. On traitera ceux-ci sans distinction, économisant ainsi en amont une opération de tri, toujours coûteuse.

De préférence, la préparation des déchets est conduite dans un broyeur granulateur, ou dans un compacteur à vis.

Si contrairement aux déchets humains ou animaux ou au sang, les déchets ont une faible humidité, par exemple s'il s'agit essentiellement de gants chirurgicaux, seringues, compresses, linge jetable souillé, etc., la préparation des déchets comprendra avantageusement une étape d'humidification ou de mouillage par de l'eau ou une solution aqueuse, afin de leur conférer une teneur globale en eau permettant une action efficace et rapide des ondes électromagnétiques. La solution aqueuse ajoutée pourra comprendre un désinfectant ou un antiseptique.

Le chauffage est réalisé par application d'ondes électromagnétiques de fréquence comprise entre 27 MHz et 9 GHz, la partie haute de la fourchette étant ce qu'on appelle communément les micro-ondes. De préférence, le chauffage est réalisé par voie diélectrique ou inductive et ce chauffage peut être également conductif ou convectif.

La manutention des déchets à l'intérieur de l'appareil est réalisée par charge individuelle discrète, c'est-à-dire par lots. Chaque lot est disposé dans un conteneur individuel.

Les déchets stérilisés seront véhiculés hors de la zone de chauffage en vue de leur transport, par exemple par éjection qui est avantageusement réalisée par voie pneumatique, hydraulique ou électrique.

On décrit également un appareil pour la mise en oeuvre du procédé selon l'invention, qui comprend :
un poste de préparation des déchets en vue de leur manutention ultérieure et pour leur traitement subséquent par chauffage électromagnétique,
un poste où les déchets sont divisés en portions homogènes et de tailles sensiblement égales,
un poste de stérilisation des déchets en lots par une source de chaleur générée par voie électromagnétique, et
un poste pour l'évacuation des déchets stérilisés hors de la zone de traitement et pour les reprendre sans exposition à l'extérieur, en vue de leur transport.

Avantageusement, l'appareil mettant en oeuvre le procédé selon l'invention comprend un disque tournant dans lequel chaque position d'arrêt correspond à autant de postes, comme indiqué plus haut, et pour le chauffage destiné à stériliser les déchets, on utilisera de préférence un appareil tel que celui qui est décrit dans l'exposé d'invention EP 0 136 453.

L'invention sera mieux comprise en référence aux figures annexées, données à titre d'exemple non limitatif, dans lesquelles, en coupe,
la figure 1 est une vue en élévation de l'appareil mettant en oeuvre le procédé selon l'invention;
la figure 2 est une vue de côté de cet appareil; et
la figure 3 est une vue de dessus de ce même appareil.

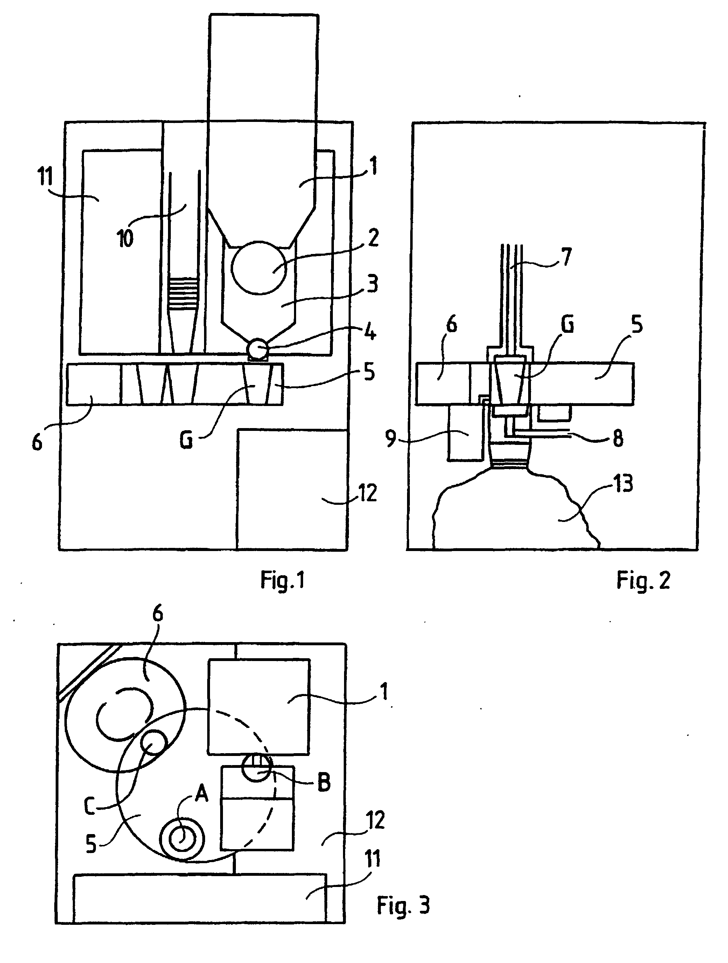

Comme on le voit sur la figure 1 qui est une vue de côté, un appareil mettant en oeuvre le procédé selon l'invention comprend une trémie d'alimentation 1 sous laquelle est situé un granulateur motorisé 2 qui débouche dans un sas tampon 3, lequel alimente à son tour un doseur motorisé 4 qui permet de remplir, par le haut, des conteneurs G.

En variante non illustrée, la trémie d'alimentation est munie d'un compacteur à déchets permettant un travail plus régulier et plus homogène du granulateur 2. Ce compacteur peut prendre par exemple la forme de deux battants articulés à leur base contre les parois de la trémie 1, au repos en appui sur les parois de celle-ci, et pouvant se rabattre l'un vers l'autre vers l'intérieur pour venir sensiblement bout-à-bout et, ce faisant, pousser et presser les déchets vers le granulateur 2.

Ces conteneurs G, reçus dans des cavités, vont donc se trouver remplis de déchets en vue de leur traitement par ondes électromagnétiques. Comme on le voit sur la figure, les conteneurs G, en forme de gobelets, proviennent d'un distributeur 10 à conteneurs et, une fois remplis par les déchets à l'aide du doseur 4 et les cavités fermées hermétiquement, ces conteneurs sont dirigés vers le guide d'ondes 6 de l'applicateur à ondes électromagnétiques. Alternativement, les conteneurs peuvent être matérialisés par un film, par exemple de polyéthylène, disposé dans chaque cavité, film que l'on remplit de déchets, que l'on ferme par soudure une fois plein. Comme dans le précédent mode de réalisation décrit, de tels conteneurs sont alors dirigés vers le guide d'ondes 6 de l'applicateur à ondes électromagnétiques, après que chaque cavité ait été fermée hermétiquement.

Pour de plus amples renseignements concernant l'applicateur à ondes électromagnétiques, on se rapportera avantageusement à l'exposé d'invention EP 0 136 453, qui est incorporé ici par voie de référence.

On remarquera sur cette figure un réservoir 11 rempli d'eau ou d'une solution aqueuse dans laquelle on pourra avoir ajouté une faible quantité de désinfectant, la solution aqueuse en question venant humecter ou mouiller les déchets granulés au niveau du doseur 4. En 12 on a représenté, sous forme schématique, les alimentations électriques de l'ensemble, ainsi qu'un compresseur.

Sur la figure 2 dans laquelle les chiffres de références indiquent les mêmes éléments que sur la figure 1, on a représenté un piston 7 qui, une fois l'opération de stérilisation par ondes électromagnétiques terminée, va éjecter le conteneur G dans un sac 13 hermétique dans lequel les déchets stérilisés pourront être transportés vers l'extérieur.

Comme on le voit sur le figure 3 dans laquelle les chiffres de références indiquent les mêmes éléments que sur les figures 1 et 2, la machine est constituée de telle sorte que les opérations s'organisent par postes autour d'une sole tournante 5 comprenant les cavités permettant de recevoir les conteneurs G. Ces postes sont disposés à 120º et chaque rotation de cet angle permet de présenter un conteneur G soit au remplissage (poste B), soit sous le magnétron, dispositif à ondes électromagnétiques pour la stérilisation (poste C), soit enfin devant le piston pour son éjection (poste A). Le poste C est entièrement logé à l'intérieur du guide d'ondes 6.

### EXEMPLE

A l'aide de l'appareil décrit ci-dessus, on a stérilisé des déchets en vrac provenant d'un bloc opératoire d'une unité de soins, à l'aide d'un appareil à ondes électromagnétiques tel que décrit dans l'exposé EP-0 136 453 précité, les déchets ayant été préalablement réduits en particules homogènes de dimensions comprises entre 1 et 5 mm. Au moment de leur alimentation dans les conteneurs, une solution aqueuse a été giclée sur ces déchets de façon à obtenir une proportion en poids d'eau relativement aux déchets dans un rapport 1 à 2.

L'application des ondes électromagnétiques, ici dans une gamme de fréquences allant de 27 MHz à 9 GHz, provoque à l'intérieur des cavités hermétiquement fermées dans laquelle sont disposés les conteneurs G une augmentation rapide de la température et de la pression pour atteindre une température supérieure à 170°C sous une pression supérieure à 4 bars. Ces conditions de température et de pression garantissent que les déchets sont effectivement stérilisés. Le chauffage fait intervenir des phénomènes diélectriques ou d'induction, et peut également faire intervenir des phénomènes de convection ou de conduction.

Le procédé selon l'invention présente l'énorme avantage d'un traitement sur site, sans transport vers l'extérieur de déchets pouvant être infectieux et donc sans risque de contaminer accidentellement l'environnement. Quand on sait la virulence et les dangers de certaines souches bactériennes ou virales pouvant infecter les malades ou sévissant dans les hôpitaux, on appréciera à sa juste mesure le progrès apporté par l'invention !

En outre, l'appareil mettant en oeuvre le procédé selon l'invention pourra éventuellement avoir des dimensions restreintes, par exemple des dimensions lui permettant de passer par les portes. Chaque unité de soin en clinique ou hôpital pourra donc s'équiper de son propre appareil, qu'elle pourra placer à sa guise aux endroits névralgiques, par exemple à proximité des blocs opératoires, d'où une très grande souplesse dans la gestion des déchets. On pourra ainsi éviter une gestion centralisée des déchets, avec tous les risques que cela peut comporter vu les volumes à traiter et les défaillances toujours possibles du matériel et des hommes.

## Revendications

1. Procédé pour le traitement des déchets médicaux et vétérinaires sur site, par stérilisation, comprenant les étapes consistant, dans l'ordre, à :
préparer les déchets en vue de leur manutention ultérieure et pour leur traitement subséquent par chauffage électromagnétique,
diviser les déchets en lots homogènes et de tailles sensiblement égales,
stériliser les déchets par lots devant une source de chaleur générée par voie électromagnétique, dans une gamme de fréquence comprise entre 27 MHz et 9 GHz et,
à véhiculer aseptiquement les déchets stérilisés hors de la zone de traitement et à reprendre ceux-ci sans exposition à l'extérieur, en vue de leur transport,
**caractérisé en ce que** le traitement à lieu à une température supérieure à 170°C et sous une pression supérieure à 4 bars.

2. Procédé selon la revendication 1, **caractérisé en ce que** la préparation des déchets comprend une étape de broyage dans un broyeur granulateur ou dans un compacteur à vis.

3. Procédé selon la revendication 1 ou 2, **caractérisé en ce que** la préparation des déchets comprend une étape d'humidification ou de mouillage par de l'eau ou une solution aqueuse.

4. Procédé selon la revendication 1 ou 3, **caractérisé en ce que** le chauffage est réalisé par application d'ondes électromagnétiques.

5. Procédé selon la revendication 4, **caractérisé en ce que** le chauffage est réalisé par voie diélectrique ou inductive et **en ce qu'**il peut également être conductif ou convectif.

6. Procédé selon la revendication 1 ou 3, **caractérisé en ce que** la manutention et la stérilisation des déchets est réalisée par lots individuels.

7. Procédé selon la revendication 6, **caractérisé en ce que** chaque lot est disposé dans un conteneur individuel.

8. Procédé selon la revendication 1 ou 3, **caractérisé en ce que** les déchets stérilisés sont véhiculés hors de la zone de chauffage en vue de leur transport par éjection pneumatique, hydraulique ou électrique.

## Claims

1. A method for the treatment of medical and veterinary waste on-site, by sterilisation, comprising the steps consisting of, in order:
• preparing the waste in view of its ulterior handling and for subsequent treatment by electromagnetic heating,
• dividing the waste in homogenous batches of substantially equal sizes,
• sterilising the waste batches using a heat source generated by electromagnetic means, at a frequency in the range of 27 MHz and 9 GHz and,
• transporting aseptically the sterilised waste out of the treatment zone and preparing it to be transported, without exposure to the outside,
• **characterised in that** the treatment takes place at a temperature greater than 170°C and under a pressure greater than 4 bar.

2. The method according to claim 1, **characterised in that** the preparation of the waste comprises a grinding step in a granulating grinder or in a screw compactor.

3. The method according to claim 1 or 2, **characterized in that** the preparation of the waste comprises a step of humidification or wetting with water or with an aqueous solution.

4. The method according to claim 1 or 3 **characterised in that** the heating is carried out by the application of electromagnetic waves.

5. The method according lo claim 4, **characterised in that** the heating is carried out by dielectric or inductive means and **in that** it can also be conductive or convective.

6. The method according to claim 1 or 3, **characterised in that** the handling and sterilisation of the waste is carried out by individual batches.

7. The method according to claim 6, **characterised in that** each batch is disposed in an individual container.

8. The method according to claim 1 or 3, **characterized in that** the sterilized wastes are conveyed out of the heating zone for transport by pneumatic, hydraulic or electrical ejection.

## Patentansprüche

1. Verfahren zur Behandlung medizinischer und veterinärer Abfälle durch Sterilisation an Ort und Stelle, die Schritte umfassend, die der Reihenfolge nach bestehen aus:
Vorbereitung der Abfälle im Hinblick auf ihre spätere Bereitstellung und für ihre nachfolgende Behandlung durch elektromagnetische Erhitzung,
Aufteilung der Abfälle in homogene Posten von im Wesentlichen gleichen Abmessungen,
postenweiser Sterilisation der Abfälle vor einer Quelle von auf elektromagnetischem Weg in einem zwischen 27 MHz und 9 GHz liegenden Frequenzbereich erzeugter Hitze und
aseptischer Beförderung der sterilisierten Abfälle aus der Behandlungszone heraus und Wiederaufnahme derselben im Hinblick auf ihren Abtransport, ohne sie der Aussenwelt auszusetzen,
**dadurch gekennzeichnet, dass** die Behandlung bei einer Temperatur oberhalb von 170 °C und unter einem Druck von mehr als 4 bar erfolgt.

2. Verfahren gemäss Anspruch 1, **dadurch gekennzeichnet, dass** die Vorbereitung der Abfälle einen Mahlschritt in einer Granuliermühle oder in einer Schraubenpresse umfasst.

3. Verfahren gemäss Anspruch 1 oder 2, **dadurch gekennzeichnet, dass** die Vorbereitung der Abfälle einen Schritt der Befeuchtung oder Durchnässung mit Wasser oder einer wässrigen Lösung umfasst.

4. Verfahren gemäss Anspruch 1 oder 3, **dadurch gekennzeichnet, dass** die Erhitzung durch Anwendung elektromagnetischer Wellen zustande kommt.

5. Verfahren gemäss Anspruch 4, **dadurch gekennzeichnet, dass** die Erhitzung auf dielektrischem oder induktivem Wege zustande kommt, und dadurch, dass sie gleichermassen auf elektrischer Leitung oder Konvektion beruhen kann.

6. Verfahren gemäss Anspruch 1 oder 3, **dadurch gekennzeichnet, dass** die Bereitstellung und die Sterilisation der Abfälle in einzelnen Posten erfolgt.

7. Verfahren gemäss Anspruch 6, **dadurch gekennzeichnet, dass** jeder Posten in einem individuellen Behälter bereitgestellt wird.

8. Verfahren gemäss Anspruch 1 oder 3, **dadurch gekennzeichnet, dass** die sterilisierten Abfälle im Hinblick auf ihren Abtransport durch Druckwasser-, Druckluft- oder elektrischen Ausstoss aus der Heizzone herausbefördert werden.
